# EUROPÄISCHE PATENTSCHRIFT

(11) **EP 1 379 184 B1**
(45) Veröffentlichungstag und Bekanntmachung des Hinweises auf die Patenterteilung: **10.01.2007**
(21) Anmeldenummer: 02735022.2
(22) Anmeldetag: 02.04.2002
(51) Int. Cl.: A61B 17/70

(54) **Fixateur zum Fixieren eines Abschnitts einer Wirbelsäule**
Fixator for fixing a section of a spinal column
Fixateur d'une section de colonne vertébrale

(30) Priorität: 06.04.2001 DE 10117426
(43) Veröffentlichungstag der Anmeldung: 14.01.2004
(73) Patentinhaber: Hahn, Michael, Dipl.-Ing., 22417 Hamburg (DE); Mauk, Rudolf, D-22844 Norderstedt (DE)
(72) Erfinder: Hahn, Michael, Dipl.-Ing., 22417 Hamburg (DE); Mauk, Rudolf, D-22844 Norderstedt (DE)
(74) Vertreter: Hansmann, Dierk
(86) Internationale Anmeldenummer: PCT/DE2002/001182
(87) Internationale Veröffentlichungsnummer: WO 2002/080787

(56) Entgegenhaltungen:
- EP-A- 0 528 177
- WO-A-02/41797
- DE-A- 4 330 837
- FR-A- 2 702 363
- FR-A- 2 745 706
- FR-A- 2 761 876
- GB-A- 2 294 394

## Beschreibung

Die Erfindung betrifft einen Fixateur zum gegenseitigen Fixieren von Wirbeln einer Wirbelsäule mit einem Aufnahmeteil, durch den sich eine Aufnahme für eine Fixierstange erstreckt und einem den Aufnahmeteil am Wirbel befestigenden Befestigungsteil.

Mittels der Erfindung läßt sich ein Verfahren zum Fixieren eines Abschnittes einer Wirbelsäule ausführen in deren zu fixierende Wirbel Pedikelschrauben eingeschraubt werden, an deren aus den Wirbeln herausragenden Enden Grundkörper befestigt werden, die mit mindestens einer Fixierstange miteinander verbunden werden.

Derartige Fixateure sind u.a. aus der DE-A-4 330 837 bekannt geworden. Sie haben sich in der praktischen Anwendung sehr gut bewährt und zeichnen sich insbesondere dadurch aus, daß sie leicht montiert und in schwierigen Anwendungsfällen gut anpaßbar sind, und zwar unabhängig davon, ob die Verbindung zwischen dem Aufnahmeteil und einer ihn tragenden Pedikelschraube dezentral oder zentral erfolgt.

Ferner ist aus der GB 229 43 94 ein Fixateur mit einem Aufnahmeteil für ein Fixierstange aus mehreren Teilstangen bekannt.

Der Oberbegriff des Anspruchs 1 basiert auf diesem Fixateur. In der praktischen Anwendung derartiger Fixateure wurde jedoch erwogen, ob eine Möglichkeit gefunden werden könne, die Fixierstange sehr unterschiedlichen Zuordnungen der einzelnen Wirbel entsprechend gestalten zu können.

Aufgabe der vorliegenden Erfindung ist es daher, einen Fixateur anzugeben, der sich unterschiedlichen räumlichen Zuordnungen benachbarter Wirbel anpassen kann.

Diese Aufgabe wird erfindungsgemäß dadurch die Merkmale des Patentanspruches 1 gelöst.

Gemäß der Erfindung besteht die Fixierstange aus mindestens zwei Teilstangen. Jede der Teilstangen besitzt jeweils vergleichsweise kleine Querschnitte. Die Teilstangen ergänzen sich hinsichtlich der von ihnen zu erwartenden Festigkeit so, daß sie gemeinsam die gleiche oder eine größere Festigkeit besitzen als nur eine sich durch die Aufnahme erstreckende Fixierstange. Darüberhinaus besitzen die Teilstangen den Vorteil, daß sie aufgrund ihres geringen Querschnittes leicht auch manuell gebogen werden können, so daß sie während der Operation sehr genau den miteinander zu verbindenen Aufnahmen entsprechend angepaßt werden können.

Gemäß einer bevorzugten Ausführungsform der Erfindung ist durch die Anzahl der jeweils eingelegten Teilstangen ein jeweils gewünschter Stabilisierungsgrad für einzelne Abschnitte der Wirbel-säule vorgebbar. Dadurch können hoch zu stabilisierende Abschnitte der Wirbelsäule mit einer größeren Anzahl von Teilstangen stabilisiert werden als weniger hoch zu stabilisierende Abschnitte.

Gemäß einer weiteren bevorzugten Ausführungsform der Erfindung ist eine bilaterale Instrumentierung als eine Kreuzverbindung zumindestens mit einem Aufnahmeteil realisierbar, das außerhalb einer Reihe von aufeinander folgenden Aufnahmeteilen angeordnet ist. Durch eine solche Kreuzverbindung läßt sich auch bei Verwendung relativ dünner Teilstangen, die jeweils zu voneinander unabhängigen Kreuzverbindungen kombiniert werden können, ein hoher Stabilisierungsgrad erreichen, ohne daß deswegen die sonst üblichen Querverbinder eingesetzt werden müssen.

Gemäß einer weiteren bevorzugten Ausführungsform der Erfindung weist die Aufnahme einen den einzulegenden Teilstangen entsprechenden Querschnitt auf. Durch eine derartige Ausbildung des Querschnittes kann eine stabile Lage der jeweiligen Teilstangen erreicht werden. Die Teilstangen können insgesamt von einer eingedrehten Stellschraube durch Druck beaufschlagt werden, ohne daß zu befürchten wäre, daß mindestens eine der Teilstangen unter dem aufgewandten Druck aus ihrer einmal eingenommenen Lage verdrängt wird und auf diese Weise zu einer Instabilität führt.

Gemäß einer weiteren bevorzugten Ausführungsform der Erfindung sind die Teilstangen durch einen Aufnahmeschlitz, der sich durch eine den Aufnahmeteil begrenzende Seitenfläche erstreckt, in die Aufnahme einlegbar. Auf diese Weise kann zunächst der Aufnahmeteil mit einer in einen Wirbel eingeschraubten Pedikelschraube verbun-den werden. Sodann kann eine gegebenenfalls in der gewünschten Weise gebogene Teilstange durch den Aufnahmeschlitz in die Aufnahme des Aufnahmeteils eingelegt werden. Sollte sich dabei herausstellen, daß die Teilstange in der in die Aufnahme eingelegten Form noch nicht in der Lage ist, zwei oder mehrere Wirbel in der gewünschten Weise zu verbinden, so kann die Teilstange leicht aus dem Aufnahmeschlitz wieder entnommen und in der erforderlichen Weise nachgerichtet werden, bis sie die gewünschte Verbindung zwischen den jeweiligen Aufnahmeteilen darstellt. Anschließend werden weitere Teilstangen ihrer jeweiligen Aufgabe gemäß gerichtet, zum Anpassen in den Aufnahmeschlitz eingelegt, gegebenenfalls dem Aufnahmeschlitz wieder entnommen und nachgerichtet, bis sie die jeweils gewünschten Wirbel optimal miteinander verbinden können.

Gemäß einer bevorzugten Ausführungsform der Erfindung verläuft der Aufnahmeschlitz parallel zur Aufnahme durch eine einem Befesti-gungsteil abgewandte Seitenfläche. Grundsätzlich stehen sämtliche Seitenflächen des Aufnahmeteils für die Herstellung des Aufnahme-schlitzes zur Verfügung. Gemäß einer weiteren bevorzugten Ausfüh-rungsform der Erfindung wird zur Einbringung des Aufnahmeschlitzes die dem Befestigungsteil gegenüberliegende Seitenfläche gewählt, da sie im Regelfall einem mit der Montage des Fixateurs befaßten Operateur am handlichsten zur Verfügung steht.

Gemäß einer weiteren bevorzugten Ausführungsform der Erfindung ist die Fixierstange in der Aufnahme durch mindestens eine sich durch den Aufnahmeteil erstreckende Stellschraube fixierbar. Dabei kann die Stellschraube grundsätzlich auch durch ein Schraubenloch eingeführt werden, das sich durch eine andere Seitenfläche erstreckt als der Aufnahmeschlitz. Gemäß einer weiteren bevorzugten Ausführungsform der Erfindung ist die Stellschraube in einer sich im Bereich des Aufnahmeschlitzes erstreckenden Gewinde-bohrung verschraubbar. Einerseits wird auf diese Weise mit Hilfe der Stellschraube ein hoher Druck auf die zu fixierende Fixierstange ausgeübt, andererseits ist auch die Stellschraube zur Sicherung der Fixierstange am besten erreichbar, wenn sich das Schraubenloch durch den Aufnahmeschlitz erstreckt, der am zweckmäßigsten durch die Seitenfläche des Aufnahmeteils verläuft, die für den Operateur am besten zu erreichen ist.

Gemäß der Erfindung weist die Aufnahme eine quer zu ihrer Längsrichtung angeordnete Schneide auf, die zwischen zwei in Längsrichtung der Aufnahme hintereinander angeordnete Stellschrauben vorgesehen ist und um die eine in die Aufnahme eingelegte Teilstange verformbar ist. Eine derartige in der Aufnahme angeordnete Schneide kann auch bei der Verwendung von Teilstangen auf einfache Weise dazu verwendet werden, die Stangen in ihrer Richtung so zu beeinflussen, daß sie genau in eine Aufnahme eines benachbarten Fixateurs einmündet.

Die bisher bekannten Verfahren zum Fixieren eines Abschnittes einer Wirbelsäule erforderten einen relativ hohen Zeitaufwand, um eine Fixierstange in eine Aufnahme eines Aufnahmeteils einzulegen und in dieser sachgerecht zu fixieren. Weiterhin besitzen diese Fixateursysteme eine einheitliche Festigkeit. Steifigkeitsvaria-tionen innerhalb einer langstreckigen Instrumentierung sind nicht möglich. Insofern bestand ein hoher Bedarf an einem Verfahren, mit dessen Hilfe das Fixieren eines Wirbels nicht nur vereinfacht, sondern auch hinsichtlich der endgültig eingenommenen Lage des Wirbels verbessert werden kann. Außerdem ist es wünschenswert, daß mit einem Fixateursystem eine den Erfordernissen angepaßte individuelle Versteifung einzelner Wirbelsäulenabschnitte erfolgen kann.

Diese Aufgabe wird mittels des erfindungsgemäßen Fixateurs dadurch gelöst, daß die Fixierstange in einen sich durch eine Seitenfläche erstreckenden Aufnahmeschlitz in eine Aufnahme eingelegt wird und in dieser durch Stellschrauben festgelegt wird. Durch diese Verfahrensschritte können sowohl gerade als auch bereits verformte Teilstangen einfach und sehr genau bezüglich der Aufnahme ausgerichtet und in dieser fixiert werden. Sollte ein Nachrichten notwendig sein, so können die Teilstangen auf einfache Weise dem Aufnahmeschlitz entnommen und in der jeweils gewünschten Weise nachgerichtet werden.

Gemäß einer weiteren bevorzugten Ausführungsform der Erfindung werden die Stellschrauben in Schraubenlöcher eingedreht, die sich durch den Aufnahmeschlitz in Richtung auf die Aufnahme erstrecken. Auf diese Weise erfolgt das Einlegen der Teilstangen und deren Fixierung mit Hilfe der Stellschrauben aus derselben Arbeitslage des die Fixierung des Wirbels vornehmenden Operateurs. Dadurch sind umständliche Umdispositionen bezüglich der Lage des Operateurs einerseits und der Fixierung der Wirbel andererseits nicht notwendig, so daß die Operation schnell und sehr genau durchgeführt werden kann.

Gemäß einer weiteren bevorzugten Ausführungsform der Erfindung wird die Fixierstange zunächst in eine die zu fixierenden Wirbel optimal gegeneinander ausgerichtete Form gebogen und anschließend in den Aufnahmeschlitz eingelegt und in-diesem festgelegt.Aufgrund der geringen Stärke einer einzelnen Teilstange ist das Biegen einfach und kann in der Regel ohne zusätzliches Instrumentarium erfolgen. Auch dadurch wird nicht nur die Zeit für die genaue Anpassung der Teilstangen in erheblicher Weise reduziert, sondern auch die Qualität der durchgeführten Arbeitsschritte vereinfacht und verbessert.

Gemäß einer weiteren bevorzugten Ausführungsform der Erfindung wird eine einer jeweils benötigten Fixierkraft entsprechenden Anzahl von Teilstangen durch den Aufnahmeschlitz in die Aufnahme eingelegt und in dieser fixiert. Diese Methode hat den Vorteil, daß die jeweilige Operation unter Verwendung eines Minumums von Teilstangen durchgeführt werden kann. Dadurch werden nicht nur Teilstangen eingespart, sondern die Steifigkeit des Fixateurs wird von Wirbel zu Wirbel dem jeweiligen Bedarf angepaßt und das Gewicht des Implantats auf ein Minumum begrenzt. Trotzdem besteht genügende Sicherheit für jeden zu versorgenden Abschnitt der ge- schädigten Wirbelsäule.

Gemäß einer weiteren bevorzugten Ausführungsform der Erfindung wird von einer Mehrzahl von in die Aufnahme eingelegten Teilstangen mindestens eine in eine von den anderen eingelegten Teilstangen abweichenden Form gebogen, bevor sie mit den anderen Teilstangen in die Aufnahmen eingelegt wird. Auf diese Weise können feinfühlend sehr genau angepaßte Konstruktionen für die jeweils geschädigte Wirbelsäule erstellt werden, die beispielsweise geeignet sind, einen Wirbel aus einer komplizierten Lage, in die er verrutscht ist, in eine der gesunden Wirbelsäule angepaßte Lage zurückzuführen und in dieser Lage zu stabilisieren.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:
Fig. 1: eine räumliche Darstellung eines Fixateurs mit drei Teilstangen und zwei Stellschrauben in Explosionsdarstellung,
Fig. 2: eine räumliche Darstellung eines Fixateurs mit einer eingelegten Teilstange und zwei ober halb des Fixateurs angeordneten Teilstangen und zwei Stellschrauben in Explosionsdarstellung,
Fig. 3: eine räumliche Darstellung eines Fixateurs mit zwei eingelegten Teilstangen und einer darüber angeordneten Teilstange und zwei Stellschrauben in Explosionsdarstellung,
Fig. 4: eine räumliche Darstellung eines Fixateurs mit drei eingelegten Teilstangen und zwei darüber angeordneten Fixiersschrauben in Explosionsdarstellung,
Fig. 5: einen Querschnitt durch einen Aufnahmeteil, drei Teilstangen und eine Stellschraube entsprechend dem Schnitt V-V in Fig.1,
Fig. 6: einen Querschnitt durch einen Aufnahmeteil, eine eingelegte Teilstange sowie zwei oberhalb des Fixateurs angeordnete Teilstangen und eine Stellschraube gemäß der Schnittlinie VI-VI in Fig. 2,
Fig. 7: einen Querschnitt durch ein Aufnahmeteil entsprechend der Schnittlinie VII-VII in Fig. 3 sowie durch eine oberhalb des Aufnahmeteils angeordnete Teilstange und eine Stellschraube,
Fig. 8: einen Querschnitt durch einen Aufnahmeteil, zwei eingelegte Teilstangen, sowie eine oberhalb des Aufnahmeteils angeordnete Teilstange und einer in den Aufnahmeteil eingeschraubten Stellschraube,
Fig. 9: einen Querschnitt durch einen Aufnahmeteil, drei eingelegte Teilstangen sowie eine in den Aufnahmeteil eingeschraubte Stellschraubegemäß der Schnittlinie IX-IX in Fig. 4, bei der allerdings die Stellschrauben außerhalb des Aufnahmeteils abgebildet sind,

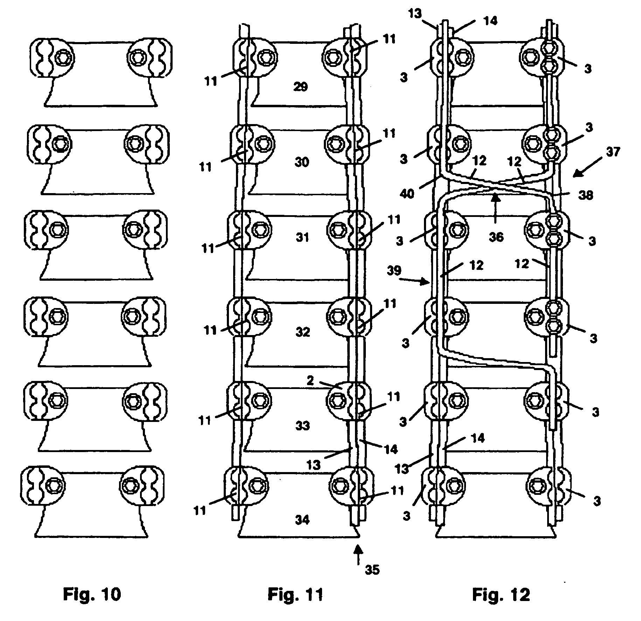

Fig. 10: eine Draufsicht auf sechs untereinander angeordnete Wirbel (stilisiert dargestellt), von denen jeder mit zwei einander gegenüberliegenden Aufnahmeteilen versehen ist, die untereinander ausgerichtet, aber unverbunden sind,
Fig. 11: eine entsprechend Figur 10 ausgerichtete Reihe von sechs Wirbeln, von denen jeder mit zwei einander gegenüberliegenden Aufnahmeteilen versehen ist, die durch Fixierstangen miteinader verbunden sind,
Fig. 12: eine entsprechend den Figuren 10 und 11 dargestellte Reihe von sechs Wirbeln, von denen jeder mit zwei einander gegenüberliegend angeordneten Aufnahmeteilen versehen ist, die paarweise durch einen Kreuzverbund miteinander verbunden sind,

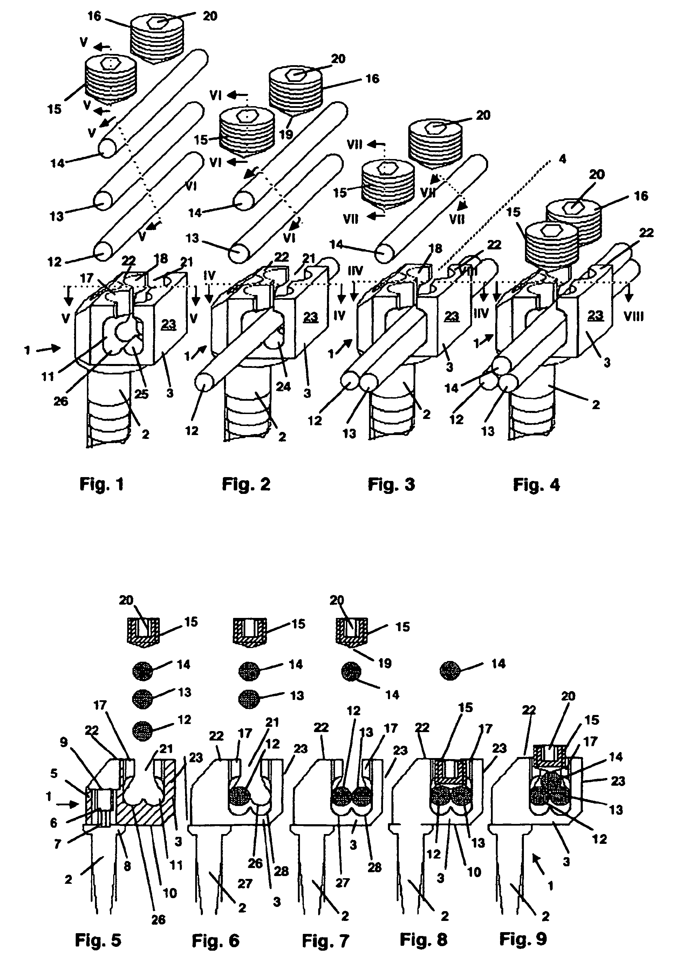

Ein Fixateur (1) besteht im wesentlichen aus einer Pedikelschraube (2) und einem Aufnahmeteil (3). Zwischen dem Aufnahmeteil (3) und der Pedikelschraube (2) besteht sehr häufig eine lösbare Verbindung, die im einzelnen nicht dargestellt ist. Sie kann zentrisch im Verlauf einer sich durch den Aufnahmeteil erstreckenden Längsachse (4) oder außerhalb dieser Längsachse (4) exzentrisch erfolgen. Eine exzentrische Lagerung ist in Fig. 5 angedeutet. Durch einen exzentrisch zur Längsachse angeordneten exzentrischen Teil (5) des Aufnahmeteils (3) erstreckt sich in Richtung auf die Pedikelschraube (2) eine Gewindebohrung (6), durch die eine nicht dargestellte Verbindungsschraube in eine Bohrung (7) hineingeschraubt wird, die in einem dem Aufnahmeteil (3) zugewandten Kopf (8) der Pedikelschraube (2) vorgesehen ist. Durch diese Gewindebohrung (6) erstreckt sich eine nicht dargestellte Verbindungsschraube bis in die Bohrung (7). Sie liegt mit ihrem nicht dargestellten Kopf auf einem Bund (9) auf, der in der Gewindebohrung (6) vorgesehen ist. Gegenüber diesem Bund (9) wird die Pedikelschraube (2) fest mit einer dem Kopf (8) zugewandten Unterfläche (10) des Aufnahmeteils (3) verschraubt.

Entlang der Längsachse (4) erstreckt sich durch das Aufnahmeteil eine Aufnahme (11), in die Teilstangen (12, 13, 14).einlegbar sind. Jede der in die Aufnahme (11) eingelegten Teilstangen (12, 13, 14) wird mit Hilfe von Sicherungsschrauben (15, 16) innerhalb der Aufnahme (11) festgelegt. Diese Sicherungsschrauben (15, 16) erstrecken sich durch Schraubenlöcher (17, 18), die in der Aufnahme (11) münden. Die in die Schraubenlöcher (17, 18) eingeschraubten Sicherungsschrauben (15, 16) beaufschlagen mit ihren den in die Aufnahme (11) eingelegten Teilstangen (12, 13, 14) zugewandten Spitzen (19) mindestens eine der in die Aufnahme (11) eingelegten Teilstangen, so daß diese eine feste Position innerhalb der Aufnahme (11) besitzen. Zum festen Anziehen der Sicherungsschrauben (15, 16) dienen profilierte Aufnahmelöcher (20), in die entsprechend gestaltete Schraubendreher (nicht dargestellt) eingesetzt werden können (Innensechskantschrauben).

Die Aufnahme (11) ist über einen Aufnahmeschlitz (21) mit einer der Seitenflächen (22, 23) verbunden. Als besonders günstig für die Lage des Aufnahmeschlitzes (21) stellt sich die der Pedikelschraube (2) abgewandte obere Seitenfläche (22) dar. Grundsätzlich kann jedoch der Aufnahmeschlitz (21) auch in der dem exzentrischen Teil (5) gegenüberliegenden seitlichen Seitenfläche (23) und auch in der Unterfläche (10) des Aufnahmeteils (3) vorgesehen sein. Durch den in der oberen Seitenfläche vorgesehenen Aufnahmeschlitz (21) können jedoch die Fixierstangen (12, 13, 14) besonders günstig in die Aufnahme (11) eingelegt werden.

Durch den Aufnahmeschlitz (21) erstrecken sich auch die Bohrungen (17, 18) in Richtung auf die Aufnahme (11). Auf diese Weise beaufschlagen die in die Schraubenlöcher (17, 18) eingeschraubten Sicherungsschrauben (15, 16), die Teilstangen (12, 13, 14) in Richtung auf eine in der Aufnahme (11) ausgebildete Aufnahmefläche (24), auf der eine oder gegebenenfalls auch zwei Teilstangen (12, 13, 14) geführt werden. Diese Aufnahmefläche (24) besitzt zwei Aufnahmemulden (25, 26), in denen jeweils eine von zwei nebeneinander angeordneten Teilstangen (12, 13) gelagert sind.

Derartige Aufnahmemulden (25) sind auch in einer sich durch die Aufnahme (11) erstreckenden Schneide (26) vorgesehen, die sich quer zur Längsachse (4) der Aufnahme (11) etwa in einem Bereich erstreckt, der zwischen den beiden Schraubenlöchern (17, 18) liegt. Diese Schneide (26) ist gegenüber einer der Unterfläche (10) zugewandten unteren Begrenzung (27, 28) der Aufnahme (11) in Richtung auf die obere Seitenfläche (22) etwas angehoben. Auf diese Weise können auf der Schneide (26) aufliegende Teilstangen (12, 13) von mindestens einer der Sicherungsschrauben (15, 16) beim Einschrauben in die Schraubenlöcher (17, 18) auf Biegung beansprucht werden. Dadurch entstehlt eine Verformung der in die Aufnahme (11) eingelegten Teilstangen (12, 13, 14), durch die eine genaue Formanpassung dieser Teilstangen (12, 13, 14) an eine Aufnahme (11) eines Wirbels (29, 30, 31, 32, 33, 34) erfolgen kann, deren Aufnahmen (11) einander in einer Reihe (35) benachbart sind. Sollte eine Aufnahme (11) eines Wirbels (32) auf einem anderen Höhenniveau liegen als die Aufnahme (11) eines benachbarten Wirbels (33), so kann je nach der gegenseitigen Lage dieser beiden Aufnahmen die Teilstange im Bereich der einen Aufnahme (11) so verformt werden, daß die Teilstange ohne Schwierigkeiten in die Aufnahme (11) eines Fixateurs (1) eingelegt werden kann, der im benachbarten Wirbel (33) mit Hilfe der entsprechenden Pedikelschraube (2) befestigt ist.

Besonders formsteif ist ein sog. Kreuzverbund (36). Dabei wird mindestens eine Teilstange (12) aus einer ersten Reihe (37) von Aufnahmeteilen (3) unmittelbar nach ihrem Austritt aus einer Aufnahme in einem Winkel (38) in Richtung auf eine parallel laufende Reihe (39) gebogen, in die sie nach Einbringung eines weiteren Winkels (40) einmündet. In entsprechender Weise wird eine Teilstange (12) der zweiten Reihe (39) in Richtung auf die erste Reihe (38) abgebogen, in die die Teilstange (12) einmündet. Dieser Kreuzverbund (36) ist sehr formstabil und daher in der Lage, die einzelnen Wirbel (29, 30, 31, 32, 33, 34) in einer gewünschten Reihenfolge zu halten, ohne daß einer der Wirbel (29, 30, 31, 32, 33, 34) aus seiner von der Wirbelsäule vorgegebenen Lage heraustreten kann.

Bei Verwendung mehrerer Teilstangen, die sich durch ein Aufnahmeteil (3) erstrecken, kann mit jeder dieser Teilstangen (12, 13, 14) ein gesonderter Kreuzverbund (36) hergestellt werden. Dabei können die einzelnen Kreuzverbunde (36) so angeordnet werden, daß sie in Richtung der ersten und der zweiten Reihe (37, 39) hintereinander angeordnet sind. Es ist jedoch auch möglich, nur eine Teilstange von jeder der beiden Reihen (37, 39) im Kreuzverbund zu führen, während weitere Teilstangen in gerader Richtung lediglich die Aufnahmeteile (3) der ersten Reihe (37) bzw. der zweiten Reihe (39) miteinander verbinden.

## Patentansprüche

1. Fixateur zum gegenseitigen Fixieren von Wirbeln einer Wirbelsäule mit einem Aufnahmeteil (3), durch den sich eine Aufnahme (11) für eine Fixierstange (12,13,14) erstreckt, eine den Aufnahmeteil (3) am Wirbel befestigendem Befestigungsteil (2), und einer Fixierstange die aus mindestens zwei Teilstangen (12, 13, 14) besteht, **dadurch gekennzeichnet, daß** das Aufnahmenteil (3) eine quer zu ihrer Längsrichtung zwischen zwei Stellschraube (15, 16) angeordnete Schneide (26) für die Verformens der Teilstangen (12, 13, 14) aufweist.

2. Fixateur nach Anspruch 1, **dadurch gekennzeichnet, daß** die Teilstangen einander etwa parallel verlaufen.

3. Fixateur nach einem der Ansprüche 1 oder 2, **dadurch gekennzeichnet, daß** die Teilstangen (12, 13, 14) durch einen Aufnahmeschlitz (21), der sich durch eine den Aufnahmeteil (3) begrenzende Seitenfläche (22) erstreckt, in die Aufnahme (11) einlegbar sind.

4. Fixateur nach Anspruch 3, **dadurch gekennzeichnet, daß** der Aufnahmeschlitz (21) parallel zur Aufnahme (11) durch eine einem Befestigungsteil abgewandte Seitenfläche (23) verläuft.

5. Fixateur nach einem der Ansprüche 3 und 4, **dadurch gekennzeichnet, daß** die Stellschraube (15, 16) in einer sich im Bereich des Aufnahmeschlitzes (21) erstreckenden Bohrung (17, 18) verschraubbar ist.

6. Fixateur nach einem der Ansprüche 1 bis 5, **dadurch gekennzeichnet, daß** jede Teilstange eine der manuellen Verformung zugängliche Festigkeit besitzt.

7. Fixateur nach einem der Ansprüche 1 bis 6, **dadurch gekennzeichnet, daß** durch die Anzahl der jeweils eingesetzten Teilstangen ein jeweils gewünschter Stabilisierungsgrad füreinzelne Abschnitte einer Wirbelsäule vorgebbar ist.

8. Fixateur nach einem der Ansprüche 1 bis 7, **dadurch gekennzeichnet, daß** eine Querverbindung zu anderen Aufnahmeteilen (3) mit mindestens einer der Teilstangen herstellbar ist.

9. Fixateur nach Anspruch 8, **dadurch gekennzeichnet, daß** mindestens eine der Teilstangen aus einer die Aufnahme (11) einseitig begrenzenden ersten Kopffläche in Richtung auf eine benachbarte Aufnahme (11) herausragt und mindestens eine weitere Teilstange als bilaterale Instrumentierung aus einer der ersten Kopffläche gegenüberliegenden, die Aufnahme (11) auf ihrer anderen Seite begrenzenden zweiten Kopffläche in Richtung auf eine dieser zweiten. Kopffläche benachbarte Aufnahme (11) herausragt und mit dieser verschraubbar ist.

10. Fixateur nach Anspruch 9, **dadurch gekennzeichnet, daß** die bilaterale Instrumentierung als ein Kreuzverband (36) zu mindestens einem Aufnahmeteil (3) realisierbar ist, das außerhalb einer Reihe (37) von aufeinanderfolgenden Aufnahmeteilen (3) angeordnet ist.

11. Fixateur nach Anspruch 10, **dadurch gekennzeichnet, daß** derKreuzverbund (36) durch eine Verbindung von mehreren Aufnahmen (11) mehrfach wiederholbar ist.

12. Fixateur nach einem der Ansprüche 1 bis 11, **dadurch gekennzeichnet, daß** jede Teilstange einen mit Unterstützung durch eine übliche Zange durchtrennbaren Querschnitt aufweist.

13. Fixateur nach einem der Ansprüche 1 bis 12, **dadurch gekennzeichnet, daß** die eine Anzahl von Teilstangen aufnehmende Aufnahme (11) eine gegenüber einer eine Fixierstange (12, 13,14) mit großem Querschnitt aufnehmenden Aufnahme kleine Baugröße aufweist.

14. Fixateur nach einem der Ansprüche 1 bis 13, **dadurch gekennzeichnet, daß** sich der Aufnahmeschlitz (21) durch eine obereSeitenfläche des Aufnahmeteils (13) erstreckt, die einer einem Wirbel (29, 30, 31, 32, 33, 34) zugewandten Unterfläche (10) des Aufnahmeteils (3) abgewandt ist.

15. Fixateur nach einem der Ansprüche 1 bis 14, **dadurch gekennzeichnet, daß** die Aufnahme (11) einen den einzulegenden Teilstangen entsprechenden Querschnitt aufweist.

16. Fixateur nach einem der Ansprüche 1 bis 15, **dadurch gekennzeichnet, daß** in der Schneide (26) mindestens eine einem Querschnitt einer Teilstange entsprechende Ausnehmung vorgesehen ist, in diejeweils eine Teilstange einlegbar ist.

## Claims

1. Fixator for fixing vertebrae of a spinal column to one another, with a receiving part (3) through which a receptacle (11) for a fixing rod (12, 13, 14) extends, a fastening part (2) which fastens the receiving part (3) to the vertebra and a fixing rod which consists of at least two partial rods (12, 13, 14), **characterised in that** the receiving part (3) comprises a sharp edge (26), disposed between two set screws (15, 16) transversely to the longitudinal direction thereof, for shaping the partial rods (12, 13, 14).

2. Fixator according to Claim 1, **characterised in that** the partial rods extend approximately parallel to one another.

3. Fixator according to either of Claims 1 and 2, **characterised in that** the partial rods (12, 13, 14) can be inserted in the receptacle (11) through a receiving slot (21) which extends through a lateral face (22) bounding the receiving part (3).

4. Fixator according to Claim 3, **characterised in that** the receiving slot (21) extends parallel to the receptacle (11) through a lateral face (23) which is remote from a fastening part.

5. Fixator according to either of Claims 3 and 4, **characterised in that** the set screw (15, 16) can be screwed into a bore (17, 18) extending in the region of the receiving slot (21).

6. Fixator according to any one of Claims 1 to 5, **characterised in that** each partial rod is of a strength which is accessible to manual shaping.

7. Fixator according to any one of Claims 1 to 6, **characterised in that** a respective desired degree of stabilisation for individual sections of a spinal column can be predetermined by the number of partial rods used in each case.

8. Fixator according to any one of Claims 1 to 7, **characterised in that** a cross connection with other receiving parts (3) can be established by at least one of the partial rods.

9. Fixator according to Claim 8, **characterised in that** at least one of the partial rods projects out of a first top face, which bounds the receptacle (11) on one side, in the direction of an adjacent receptacle (11), and at least one further partial rod, as a bilateral instrumentation, projects out of a second top face, which lies opposite the first top face and bounds the receptacle (11) on its other side, in the direction of a receptacle (11) adjacent to this second top face and can be screwed to this.

10. Fixator according to Claim 9, **characterised in that** the bilateral instrumentation can be implemented as a cross brace (36) to at least one receiving part (3) which is disposed outside of a row (37) of successive receiving parts (3).

11. Fixator according to Claim 10, **characterised in that** the cross brace (36) can be repeated several times through a connection of a plurality of receptacles (11).

12. Fixator according to any one of Claims 1 to 11, **characterised in that** each partial rod has a cross section which can be severed with the assistance of a conventional pair of nippers.

13. Fixator according to any one of Claims 1 to 12, **characterised in that** the receptacle (11) receiving a number of partial rods is of a small overall size with respect to a receptacle receiving a fixing rod (12, 13, 14) of a large cross section.

14. Fixator according to any one of Claims 1 to 13, **characterised in that** the receiving slot (21) extends through a top lateral face of the receiving part (13) which is remote from an underface (10) of the receiving part (3) which faces a vertebra (29, 30, 31, 32, 33, 34).

15. Fixator according to any one of Claims 1 to 14, **characterised in that** the receptacle (11) has a cross section which corresponds to the partial rods which are to be inserted.

16. Fixator according to any one of Claims 1 to 15, **characterised in that** at least one recess corresponding to a cross section of a partial rod is provided in the sharp edge (26), in which recess a respective partial rod can be inserted.

## Revendications

1. Fixateur destiné à la fixation mutuelle de vertèbres d'une colonne vertébrale comprenant une pièce de réception (3) à travers laquelle s'étend un logement (11) pour une tige de fixation (12, 13, 14); : une pièce de fixation (2) fixant la pièce de réception (3) à la vertèbre et une tige de fixation qui est constituée d'au moins deux tiges partielles (12, 13), **caractérisé en ce que** le logement présente une arête (26) disposée transversalement à son étendue longitudinale entre deux vis de serrage (15, 16) pour la déformation des tiges partielles (12, 13, 14).

2. Fixateur selon la revendication 1, **caractérisé en ce que** les tiges partielles s'étendent de manière approximativement parallèle les unes aux autres.

3. Fixateur selon l'une des revendications 1 ou 2, **caractérisé en ce que** les tiges partielles (12, 13, 14) peuvent être placées dans le logement (11) par une fente de réception (21) qui s'étend à travers la surface latérale (22) limitant la pièce de réception (3).

4. Fixateur selon la revendication 3, **caractérisé en ce que** la fente de réception (21) court parallèlement au logement (11) à travers une surface latérale (23) opposée à une pièce de fixation.

5. Fixateur selon l'une des revendications 3 et 4, **caractérisé en ce que** la vis de serrage (15, 16) peut être vissée dans un trou (17, 18) s'étendant dans la zone de la fente de réception (21).

6. Fixateur selon l'une des revendications 1 à 5, **caractérisé en ce que** chaque tige partielle a une résistance accessible à la déformation manuelle.

7. Fixateur selon l'une des revendications 1 à 6, **caractérisé en ce qu'**un degré de stabilisation souhaité de sections individuelles d'une colonne vertébrale peut être prédéfini par le nombre des tiges partielles respectivement mises en place.

8. Fixateur selon l'une des revendications 1 à 7, **caractérisé en ce qu'**une connexion transversale avec d'autres pièces de réception (3) peut être réalisée avec au moins une des tiges partielles.

9. Fixateur selon la revendication 8, **caractérisé en ce qu'**au moins une des tiges partielles fait saillie d'une première surface de tête limitant le logement (11) d'un côté en direction d'un logement (11) voisin et au moins une autre tige partielle fait saillie, en tant qu'instrumentation bilatérale, d'une deuxième surface de tête opposée à la première surface de tête, limitant le logement (11) sur son autre côté, en direction d'un logement (11) voisin de cette deuxième surface de tête et peut être vissée à celui-ci.

10. Fixateur selon la revendication 9, **caractérisé en ce que** l'instrumentation bilatérale peut être réalisée comme une liaison croisée (36) par rapport à au moins une pièce de réception (3) qui est disposée en dehors d'une rangée (37) de pièces de réception (3) successives.

11. Fixateur selon la revendication 10, **caractérisé en ce que** la liaison croisée (36) peut être reproduite plusieurs fois par une connexion de plusieurs logements (11).

12. Fixateur selon l'une des revendications 1 à 11, **caractérisé en ce que** chaque tige partielle présente une section transversale sectionnable à l'aide d'une pince usuelle.

13. Fixateur selon l'une des revendications 1 à 12, **caractérisé en ce que** le logement (11) logeant un nombre de tiges partielles présente une petite taille par rapport à un logement logeant une tige de fixation (12, 13, 14) avec une grande section transversale.

14. Fixateur selon l'une des revendications 1 à 13, **caractérisé en ce que** la fente de réception (21) s'étend à travers une surface latérale supérieure de la pièce de réception (13) qui est opposée à une surface inférieure (10) de la pièce de réception (3) qui est orientée vers une vertèbre (29, 30, 31, 32, 33, 34).

15. Fixateur selon l'une des revendications 1 à 14, **caractérisé en ce que** le logement (11) présente une section transversale correspondant aux tiges partielles à mettre en place:

16. Fixateur selon l'une des revendications 1 à 15, **caractérisé en ce qu'**il est prévu dans l'arête (26) au moins un creux correspondant à une section transversale d'une tige partielle dans lequel une tige partielle peut chaque fois être mise en place.
